# EUROPÄISCHE PATENTANMELDUNG

(11) **EP 0 878 544 A1**
(43) Veröffentlichungstag der Anmeldung: **18.11.1998**
(21) Anmeldenummer: 98250131.4
(22) Anmeldetag: 29.01.1991
(51) Int. Cl.: C12N 15/12, C12N 15/70, C12N 1/21, C12N 5/10, C07K 14/47, C07K 16/18, A61K 38/17, A61K 39/395, G01N 33/53

(54) **Epididymis-spezifische DNA-Sequenzen und deren Verwendung**

(30) Priorität: 01.02.1990 DE 4002981; 30.11.1990 DE 4038189
(62) Teilanmeldung aus: 91250021.2
(71) Anmelder: IHF INSTITUT FÜR HORMON- UND FORTPFLANZUNGSFORSCHUNG GmbH, 22529 Hamburg (DE)
(72) Erfinder: Ivell, Richard, Dr., 20257 Hamburg (DE); Kirchhoff, Christiane, 24582 Wattenbek (DE)
(74) Vertreter: UEXKÜLL & STOLBERG

(57) **Zusammenfassung**

Es werden Epididymis-spezifische Polypeptide des Menschen, sowie für diese Polypeptide kodierende Nukleotidsequenzen beschrieben. Die erfindungsgemäßen Polypeptide stehen in engem Zusammenhang mit der Spermienreifung. Sie sind demgemäß zur Diagnose und Behandlung männlicher Infertilität geeignet. Die Erfindung betrifft ferner Antikörper gegen die erfindungsgemäßen Polypeptide sowie deren Verwendung bei der Diagnose und Behandlung männlicher Fertilität.

## Beschreibung

Die Erfindung betrifft neue, Epididymis-spezifische DNA-Sequenzen (Seq. ID-Nrn. 1 bis 5) sowie ferner von diesen abgeleitete Polypeptide und deren Verwendung zur Herstellung von Arzneimitteln zur Diagnose und Behandlung der männlichen Infertilität.

Derzeit sind ca. 15% aller Paare in der Bundesrepublik Deutschland ungewollt kinderlos und ihr Anteil nimmt ständig zu. Die Ursachen für die Unfruchtbarkeit liegen zu gleichen Teilen bei Mann und Frau. Während jedoch die Ursachen bei der Frau weitgehend diagnostizierbar sind, können beim Mann nur in ca. 30% der Fälle organische Ursachen festgestellt werden. Etwa ein Drittel der verbleibenden 70% lassen sich auf Oligospermie unbekannten Ursprungs zurückführen, während bei den restlichen Fällen nach dem bisherigen Wissensstand keine organisch-biochemisch manifestierbaren Ursachen vorliegen.

Bei der Reifung der Spermien nimmt der Epididymis (Nebenhoden) eine Schlüsselrolle ein. Er besteht beim Menschen aus einem einzigen 5 m langen Kanal, der sehr stark mäanderartig aufgewunden ist. Die im Hoden gebildeten, noch unreifen Spermien werden zum Epididymis transportiert und während einer 2 bis 3 Tage dauernden Passage einem Reifungsprozeß unterzogen, im Verlaufe dessen sie u.a. ihre Beweglichkeit erlangen.

Es ist demgemäß davon auszugehen, daß männliche Unfruchtbarkeit in zahlreichen Fällen auf Störungen der im Epididymis ablaufenden Reifungsprozesse beruht.

Obwohl die anatomische Feinstruktur des humanen Epididymis makroskopisch, mikroskopisch und elektronenmikroskopisch sehr gut beschrieben ist (vgl. A.F. Holstein: Morphologische Studien am Nebenhoden des Menschen, zwanglose Abhandlungen aus dem Gebiet der normalen und pathologischen Anatomie, **20**, 1-91 (1969)), sind dessen Proteinprodukte mit wenigen Ausnahmen (vgl. beispielsweise Tezon et al., Immunochemical localization of secretory antigens in the human epididymis and their association with spermatozon, Biology of Reproduktion, **32**, 591-597 (1985)) nur wenig erforscht. Fast alle Kenntnisse über dieses Organ stammen aus Arbeiten an Ratten, Mäusen, Hamstern, Ebern, Bullen oder gelegentlich Affen, wobei die Tier-spezifischen Unterschiede bekanntermaßen groß sind.

Die an verschiedenen Tierarten gewonnenen Ergebnisse zur Spermienreifung beim Durchtritt durch den Epididymis lassen sich wie folgt zusammenfassen (vgl. T.G. Cooper: The Epididymis, Sperm Maturation and Fertilisation, Springer Verlag, Berlin, (1986)):
(a) Begeißelung und Hyperaktivierung der Spermien.
(b) Kapazitation, d.h. die Bereitschaft, die Akrosomenreaktion durchzuführen, wobei Dekapazitationsfaktoren, bei denen es sich vermutlich um epididymale Polypeptide handelt, eine regelnde Rolle spielen.
(c) Veränderung der Oberflächenantigene der Spermien, um die Bindung zwischen Spermien und Eizelle zu begünstigen.
(d) Veränderung der Spermienmembran, um die Fusion mit dem Ei zu ermöglichen.

Das diese Prozesse auslösende Stoffwechselgeschehen im Epididymis ist selbst bei den untersuchten Tieren weitgehend ungeklärt. Es konnte jedoch anhand von Gelelektrophoresen gezeigt werden, daß im Epididymis einige Polypeptide zu der aus der Rete Testis stammenden Seminalflüssigkeit hinzukommen. Unter diesen sind bei der Ratte die Polypeptide (a) bis (e) von Cooper (a.a.O., Tabelle 20), in denen das sogenannte "acidic epididymal glycoprotein (AEG)" (Lea et al., (1978)) bzw. die Polypeptide B bis E von Brooks und Mitarbeiter (D.E. Brooks und J. Higgins, Characterization and androgen-dependence of proteins associated with lunimal fluid and spermatozoa in the rat epididymis, Journal of Reproduction and Fertility, **59**, 363-375, (1980)) enthalten sind. Die Ergebnisse dieser Modelle weisen darauf hin, daß die Polypeptide unter Androgeneinfluß stehen.

Die aus Tierversuchen gewonnen Ergebnisse lassen sich jedoch aufgrund der hohen Gewebe- und Spezies-Spezifität nicht auf den Menschen übertragen. Beispielsweise wurden die bei der Ratte wesentlichen epididymalen Polypeptide oder die sie kodierenden mRNA's in keinem anderen Gewebe und, abgesehen von der Maus, in keiner anderen Spezies gefunden (D.E. Brooks et al., Europ. J. Biochem., **161**, 13-18 (1986); J. Biolog. Chem., **261**, 4956-4961, (1986)).

Es ist daher Aufgabe der Erfindung, Mittel zu schaffen, mit deren Hilfe Störungen des Proteinstoffwechsels im Epididymis beim Menschen - und insbesondere männliche Infertilität - diagnostiziert und gegebenenfalls therapiert werden können.

Zur Lösung der Aufgabe werden die erfindungsgemäßen Epididymis-spezifischen Polypeptide humanen Ursprungs sowie Fragmente derselben und allelische Varianten mit gleicher Immunogenität vorgeschlagen.

Zur Verwendung bei der Diagnose sind ferner die DNA-Sequenzen gemäß Sequenzprotokoll (Seq. ID-Nrn. 1 bis 5) sowie syngene Sequenzen oder Fragmente derselben, aber auch solche Nukleotidsequenzen, welche mit den zuvor genannten hybridisieren - insbesondere als Sonden - geeignet, wobei durch die Degeneration des genetischen Codes verursachte Abweichungen erfindungsgemäß eingeschlossen sind.

Die Isolierung und Identifikation Epididymis-spezifischer Polypeptide nach konventionellen Verfahren ist wegen der geringen Mengen des zur Verfügung stehenden Gewebes nicht möglich.

Im Rahmen der vorliegenden Erfindung ist es nunmehr erstmals gelungen, spezifische Sekretproteine des menschlichen Nebenhodens nachzuweisen, zu charakterisieren und ihre Herstellung mittels Verfahren auf der Basis rekombinanter DNA-Technologie oder chemischer Synthese zu ermöglichen.

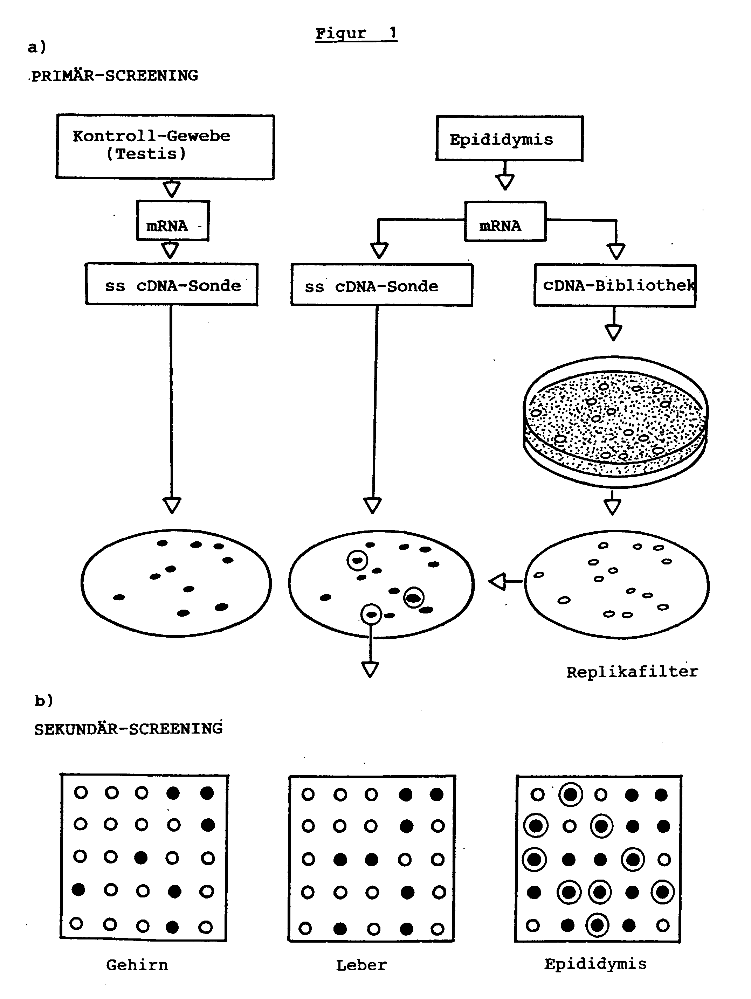

Zur Identifizierung der erfindungsgemäßen Polypeptide wurde zunächst eine cDNA-Bibliothek aus epididymaler mRNA in Lambda gtll (Clontech, California) erstellt, sodann wurden die Epididymis-spezifischen Rekombinanten in einer komplexen Folge von differentiellen Hybridisierungsschritten selektiert (vgl. Figur 1) und anschließend in dem bakteriellen Plasmid pSB (Stratagene, California, USA) subkloniert und charakterisiert.

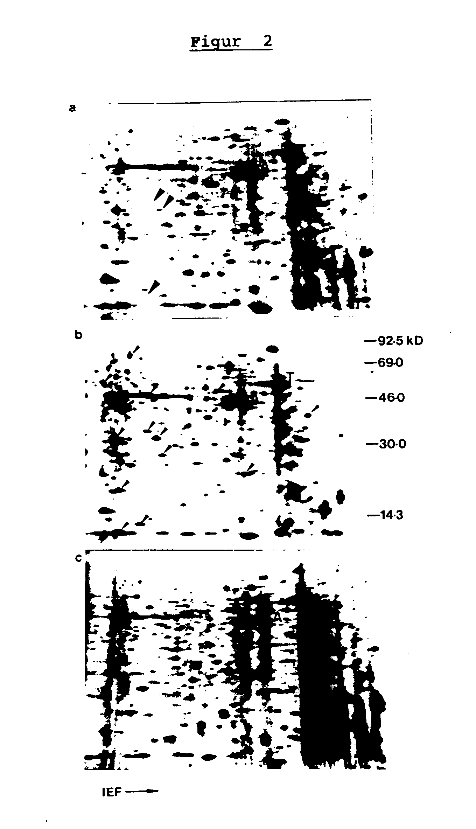

Das differentielle Screening zielte darauf ab, Klone von mRNA's zu isolieren, welche im Epididymis-Gewebe nicht aber im Gewebe anderer und funktionsverwandter Organe vorhanden sind oder dort nur in wesentlich reduzierter Kopienzahl auftreten. Bei dem erfindungsgemäß eingesetzten Verfahren diente im ersten Schritt menschliches Hodengewebe als Vergleichsmaterial. Da einige der als Ausgangsmaterial verwendeten Gewebeproben von orchiektomierten Männern stammten, die mit verschiedenen Medikamenten behandelt worden waren, mußte zunächst überprüft werden, ob und in welchem Umfang die Medikamentation Einfluß auf das jeweilige Polypeptidmuster ausübt. Es konnte jedoch an 7 Patienten unterschiedlichen Alters und unterschiedlicher Medikamentation gezeigt werden (vgl. Beispiel 2 und Figur 2a bis c), daß die Muster durch vorherige Verabreichung von Medikamenten nur unwesentlich beeinflußt werden und daß auch die individuellen Abweichungen gering sind.

Wie in Figur 1 schematisch dargestellt ist, erfolgte das Screening erfindungsgemäß in einer primären und einer sekundären Stufe, wobei in der primären Stufe durch Kreuzhybridisieren mit Testis-mRNA potentiell Epididymis-spezifische Klone isoliert wurden und diese in der sekundären Stufe durch Kreuzhybridisieren mit mRNA aus Gehirn und Leber weiter selektioniert wurden (vgl. Beispiel 4 und Figuren 3 und 4).

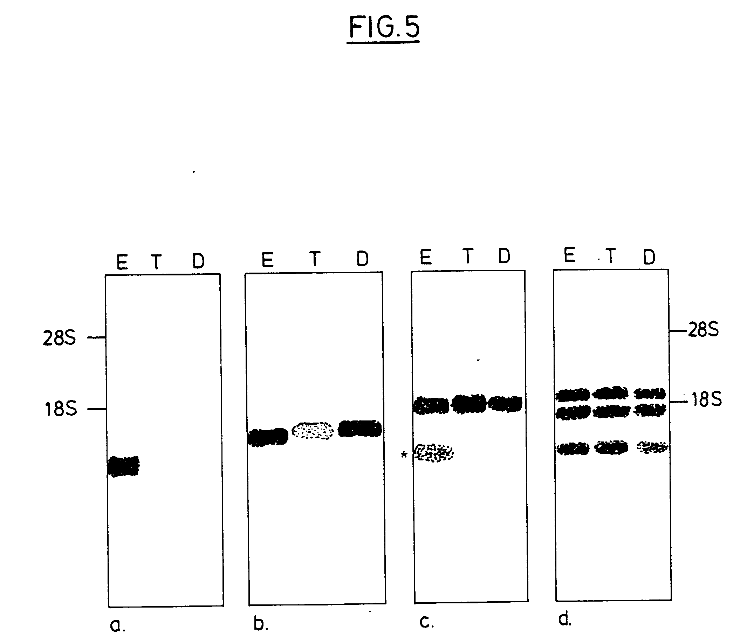

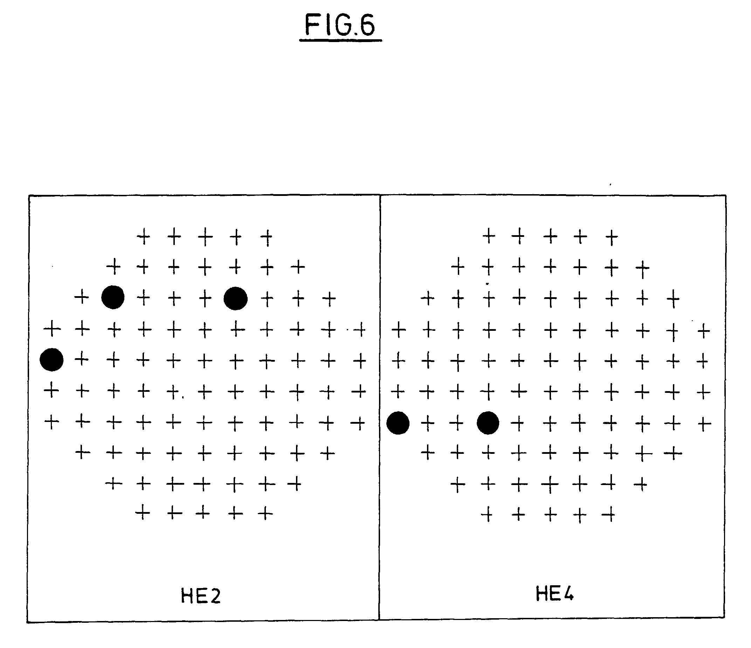

Durch nachfolgende Northern-Blot-Analyse gegen Gesamt-mRNA aus Hoden und menschlicher Decidua konnte die Zahl der in Betracht kommenden Klone weiter eingeengt werden und diese schließlich fünf unabhängigen cDNA-Familien zugeordnet werden (vgl. HE 1 bis HE 5, Beispiel 4 und Figuren 5 und 6).

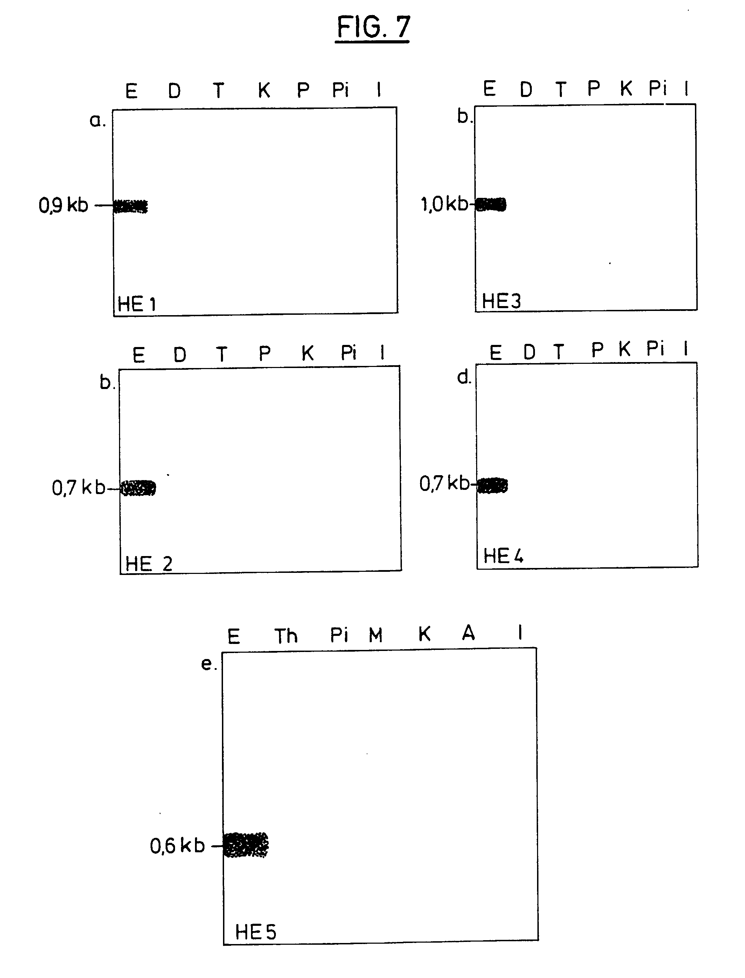

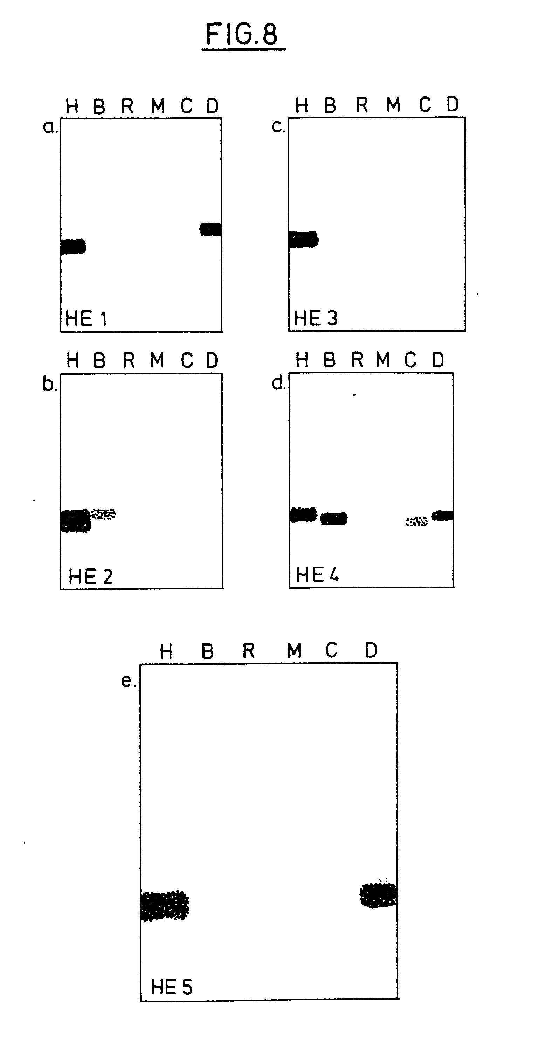

Die erfindungsgemäß gewonnenen Ergebnisse zeigen, daß die diesen fünf Klonen zugrunde liegenden mRNA's gar nicht oder nur sehr schwach in anderen menschlichen Geweben synthetisiert werden (vgl. Figur 7); es handelt sich demgemäß um Epididymis-spezifische Moleküle, die zugleich überwiegend Spezies-spezifisch sind (vgl. Fig. 8). Übereinstimmungen zeigten sich mit HE 1, HE 4 und HE 5 nur beim Hund, wobei HE 4 auch beim Rind exprimiert wird.

Die Figuren 5 bis 8 sind schematische Zeichnungen der jeweils zugrundeliegenden Autoradiogramme.

Die DNA-Sequenzen der erfindungsgemäßen cDNA-Klone HE 1 bis HE 5 (Seq. ID-Nrn. 1 bis 5) wurden wie in Beispiel 7 angegeben analysiert. Die Ergebnisse sind im Sequenzprotokoll wiedergegeben; die für HE 3 angegebene DNA-Sequenz (Seq. ID-Nr. 3) stellt ein Fragment dar, welches bezogen auf die genomische DNA stromabwärts der kodierenden Region lokalisiert ist. Obgleich das Fragment HE 3 (Seq. ID-Nr. 3) nicht als Basis für die Herstellung des entsprechenden Polypeptids dienen kann, so bildet es als Sonde für den Nachweis der entsprechenden mRNA des Klons HE 3 ein wertvolles diagnostisches Instrument.

Die gefundenen Sequenzen (Seq. ID-Nrn. 1 bis 5) wurden auf Homologie zu den in verschiedenen internationalen Datenbanken (NIH-Genbank database, EMBL, PIR) gespeicherten Gensequenzen untersucht. Dabei zeigte es sich, daß es sich um bisher unbekannte Gene handelt.

Das Vorhandensein tentativer Signalpeptidsequenzen in HE 1, HE 2, HE 4 und HE 5 (Seq. ID-Nrn. 1, 2, 4 und 5) zeigt, daß es sich um Gene handelt, welche für Sekretpolypeptide kodieren.

Die Klone HE 1, HE 2, HE 4 und HE 5 können nach herkömmlichen Verfahren über geeignete Vektoren in pro- oder eukaryontische Wirtszellen transferiert und dort als Proteine exprimiert werden.

Erfindungsgemäß sind alle jene DNA-Sequenzen geeignet und eingeschlossen, die nach Transformation geeigneter pro- oder eukaryontischer Wirtszellen die Gewinnung von Nukleinsäuren zur Verwendung als Diagnostika und/oder die Expression von Polypeptiden gewährleisten, welche zumindest einen Teil der jeweiligen Primärstruktur und eine oder mehrere der biologischen oder immunogenen Eigenschaften der erfindungsgemäßen Polypeptide aufweisen. Diese Sequenzen in einzel- oder doppelsträngiger Form schließen insbesondere ein:
a) Die im Sequenzprotokoll unter den Seq. ID-Nrn. 1 bis 5 angegebenen Nukleotidsequenzen oder syngene Sequenzen oder Fragmente derselben sowie deren komplementäre Sequenzen;
b) DNA-Sequenzen, die mit den Protein-kodierenden Bereichen der unter a) angegebenen Nukleotidsequenzen oder mit der im Sequenzprotokoll unter der Seq. ID-Nr. 3 angegebenen Sequenz oder Fragmenten derselben hybridisieren, beispielsweise unter den in den Beispielen 3 oder 4 angegebenen Hybridisierungsbedingungen;
c) DNA-Sequenzen, die mit Ausnahme der durch die Degeneration des genetischen Codes verursachten Abweichungen, mit den unter a) und b) genannten Sequenzen hybridisieren.

Der Begriff "syngene Sequenz" umfaßt alle Sequenzen, die sich von dem gleichen oder homologen Gen ableiten und für die Polypeptide im Sinne der Erfindung kodieren bzw. zur Herstellung von Sonden verwendbar sind.

Die Erfindung umfaßt ferner natürlich vorkommende allelische Variationen der erfindungsgemäßen Polypeptide oder Fragmente derartiger Allelvariationen, wobei sich die verschiedenen allelischen Formen sowohl jeweils voneinander, als auch von den im Sequenzprotokoll unter den Seq. ID-Nrn. 1, 2, 4 und 5 angegebenen Aminosäuresequenzen hinsichtlich der jeweiligen Sequenzlänge als auch in Bezug auf Deletionen, Substitutionen, Insertionen oder Additionen von Aminosäuren unterscheiden können.

Als geeignete Vektoren für prokaryontische Wirtszellen kommen beispielsweise Plasmide der pET-Serie (Rosenburg et al., "Vectors for selective expression of cloned DNAs by T7 RNA Polymerase", Gene, 56, S. 125-135 (1987)), der pGEX-Serie (Pharmacia, Freiburg) und der pRIT-Serie (Pharmacia, Freiburg) in Betracht. Beispiele für prokaryontische Wirtszellen sind die üblichen Laborstämme von Escherichia coli K12 sowie die Stämme BL21 (DE3) und LE392.

Beispiele geeigneter Vektorsysteme für eukaryontische Wirtszellen sind im Falle von Säuger-Zellen SV40 Viren, Polyoma-Viren, Adenoviren, verschiedene Retroviren, Papillomaviren (P.W.J. Rigby, "Expression of cloned genes in eucaryotic cells using vector systems derived from viral replicons", Genetic Engineering, Bd. 3, S. 84-141 (1982)), sowie Vaccinia-Viren (Mackett et al., "The construction and characterization of Vaccinia Virus recombinants expressing foreign genes", DNA Cloning, Bd. II, (1985), IRL Press, Oxford), und Derivate derselben sowie solche Plasmide, die Teile viraler Gene aufweisen (z.B. pSV2) und als "Shuttle-Vektoren" eingesetzt werden können (P.W.J. Rigby, a.a.O.). Als Vektoren für Insekten-Zellen kommen beispielsweise pJVETL-Bakuloviren (Invitrogen, San Diego, Calif., USA) in Betracht, während für Hefe-Zellen z.B. pJP31, YEp- und YIp-Plasmide (Carter et al., "Expression and secretion of foreign genes in Yeast", DNA Cloning, Bd. III, (1987), IRL Press, Oxford) verwendet werden können.

Geeignete Säuger-Zellen sind z.B. COS-Zellen, CHO-Zellen, AtT20-Zellen und NIH3T3-Zellen (Rigby, a.a.O., Mackett et al., a.a.O.), während geeignete Insekten-Zellen z.B. Sf9 (Invitrogen, San Diego, Calif., USA) sind. Geeignete Hefe-Zellen sind z.B. X4003-5B (Carter, a.a.O.).

Ferner können die den Klonen HE 1, HE 2, HE 4 und HE 5 entsprechenden Polypeptide nach bekannten Verfahren chemisch synthetisiert werden (J.M. Stewart, "Synthesis and use of neuropeptides", Neuroendocrine Peptide Methodology, ed. P.M. Conn, Academic Press, New York, S. 815-844 (1989))). Das gleiche gilt für Polypeptide oder Peptidepitope mit gleicher Immunogenität, die von Fragmenten oder syngenen Sequenzen der erfindungsgen:äßen DNA-Sequenzen (Seq. ID-Nrn. 1, 2, 4 und 5) kodiert sind.

Die erfindungsgemäßen Nukleotidsequenzen (Seq. ID-Nrn. 1 bis 5) einschließlich ihrer komplementären Sequenzen, ihre Expressionsprodukte sowie auf deren Basis hergestellte Antikörper bieten erstmals die Möglichkeit, Störungen im Proteinstoffwechsel des Epididymis zu diagnostizieren und gegebenenfalls zu therapieren.

So können beispielsweise die cDNA-Sequenzen oder deren komplementäre Sequenzen oder Fragmente derselben mit Markern versehen und als Sonde zur in situ-Hybridisierung bei der Gewebediagnostik von Biopsieproben oder Dünnschnitten verwendet werden, um den physiologischen Zustand des Gewebes hinsichtlich vorhandener Proteine zu bestimmen.

Ferner sind in diesem Zusammenhang markierte Antikörper einsetzbar, die von den erfindungsgemäßen Polypeptiden oder Fragmenten derselben induziert und in der Lage sind, eine Immunreaktion mit den entsprechenden Gewebeproteinen einzugehen.

Die auf der Basis der Sequenzen in prokaryontischen oder eukaryontischen Wirtszellen exprimierten oder synthetisch hergestellten Polypeptide sowie deren allelische Varianten mit gleicher Immunogenität oder Fragmente derselben können ferner in markierter oder unmarkierter Form als Antigene zur Identifizierung von Autoantikörpern in den Seren infertiler Männer dienen. Diese Möglichkeit ist von besonderer Bedeutung, da vermutet wird, daß die Infertilität in einem großen Teil der Fälle auf das Vorhandensein von Autoantikörpern gegen wesentliche Komponenten des Fortpflanzungssystems zurückzuführen ist. Die bisher zur Verfügung stehenden Testmethoden messen jedoch nur Antikörper, die gegen einige Sperm-Oberflächen-Antigene gerichtet sind, wobei ein ausreichend hoher Titer der Antikörper vorhanden sein muß, um die Sperm-Agglutination erfolgen zu lassen. Es wird jedoch angenommen, daß Antikörper in weitaus niedrigeren Titern vorhanden sein und Infertilität verursachen können. Diese könnten mit den erfindungsgemäß hergestellten reinen Polypeptiden als Antigene erfaßt werden.

Ferner können die erfindungsgemäßen Polypeptide oder Fragmente derselben mit gleicher biologischer Wirksamkeit für die in vitro Behandlung von Spermien infertiler Männer zum Einsatz kommen.

Polyklonale und monoklonale Antikörper zur Verwendung in immunologischen Nachweisverfahren können mit Hilfe der erfindungsgemäßen hochreinen Polypeptide auf bekannte Weise hergestellt werden. Derartige Antikörper lassen sich auf der Basis der vollständigen Polypeptide sowie auf der Basis von Fragmenten und allelischen Varianten derselben herstellen, soweit diese die gleiche Immunogenität besitzen (vgl. Beispiele 8, 9).

Ausgehend von der cDNA-Sequenz bieten sich hinsichtlich der Gewinnung von Antigenen zur Herstellung von Antikörpern zwei unterschiedliche Verfahren an.

Zum einen läßt sich mit Hilfe eines Computers eine potentiell immunogene Region der jeweils interessierenden, von der entsprechenden cDNA-Sequenz abgeleiteten Proteinsequenz auswählen, welche einerseits relativ hydrophil ist und damit an der Außenseite des Proteinmoleküls liegt, andererseits aber nicht durch Bildung von Cystein-Doppelbrücken oder möglichen Glykosilierungsstellen in ihrer sterischen Konformation gestört wird. Dieser Peptidbereich wird anschließend gegebenenfalls zusammen mit flankierenden Aminosäuren synthetisiert, nachfolgend an Trägersubstanzen gekoppelt und als Immunogen für die Induktion von Antikörpern eingesetzt (vgl. Beispiel 9).

Alternativ läßt sich beispielsweise das interessierende cDNA-Insert insgesamt oder aber ein großer Bereich davon in einen geeigneten Expressions-Plasmidvektor umklonieren. Nach anschließender Transformation in geeignete Bakterien erlauben diese Vektoren eine induzierbare Expression des von dem cDNA-Molekül kodierten Proteins. Das auf diese Weise hergestellte bakteriogene Protein oder Proteinfragment kann nach Aufreinigung aus dem Bakterienextrakt direkt als Antigen für eine Immunisierung zur Induktion von Antikörpern verwendet werden (vgl. Beispiel 8).

Die Antikörper können mit einem Marker wie z.B. einem fluoreszierenden Molekül (Fluorophor) versehen und beispielsweise bei Spermproben eingesetzt werden, um bei Infertilität das Vorhandensein von Nebenhoden-spezifischen Proteinen auf der Spermoberfläche mit Hilfe der Immunfluoreszenz zu bestimmen.

Weiterhin bietet sich die Möglichkeit, die Antikörper in einem Immunassay mit Seminalplasmaproben einzusetzen, um die Funktion des Nebenhodens zu überprüfen.

Sie können ferner zur Isolierung der entsprechenden Polypeptide aus Körperflüssigkeiten beispielsweise mittels Säulenchromatographie dienen. Die Erfindung umfaßt demgemäß auch derartige aus natürlichen Quellen isolierte Polypeptide.

Schließlich können die erfindungsgemäßen HE-Proteine und gegen diese gerichtete Antikörper zur Immunsterilisierung verschiedener Säuger eingesetzt werden.

Da die HE-Proteine ausschließlich in männlichen Organen synthetisiert werden, sind sie insbesondere zur Anwendung im weiblichen Organismus geeignet. Die dort gegen die Fremdproteine erzeugten Antikörper werden nur an Spermien haftende Nebenhodenproteine im weiblichen Genitaltrakt erkennen und zu deren Agglutination bzw. Inaktivierung, nicht aber zu unerwünschten Nebenwirkungen durch Reaktionen mit Proteinen des weiblichen Organismus führen.

Eine Verhütungsimmunisierung im männlichen Organismus durch Verabreichung von HE-Proteinen zur Auslösung einer Autoimmunantwort, die sich im Bereich des Nebenhodens in Form einer Inaktivierung der ihn durchwandernden Spermien auswirkt, stellt eine weitere Anwendungsform dar.

Aufgrund der Kreuzhomologie der Klone HE 1, HE 4 und HE 5 mit dem Hund wird zudem erfindungsgemäß ein wertvolles Mittel zum Sterilisieren von Hunden geschaffen.

### Beispiel 2

### In vitro-Translation und Charakterisierung der Produkte durch zweidimensionale Gelelektrophorese

0,5 bis 1 µg der Poly(A)⁺RNA von Nebenhoden und Hoden aus Beispiel 1 wurden jeweils in einem zellfreien System eines Retikulozyten-Lysats aus Kaninchen (New England Nuclear (NEN), Dreieich, BRD) in vitro-translatiert. Die Synthese wurde in Gegenwart von (³⁵S)-Methionin spezifische Aktivität >1000 Ci/mmol) durchgeführt. Die resultierenden Proteinprodukte wurden anschließend in einem zweidimensionalen Gel elektrophoretisch aufgetrennt (vgl. P.H. O'Farrel, High resolution two-dimensional electrophoresis of proteins, J.Biol. Chem., **250**, 4007-4021 (1975)). Für die erste Dimension wurde jeweils eine Menge von 350 000 cpm der mit (³⁵S)-Methionin markierten Translationsansätze auf das Gel aufgetragen und bei einer angelegten Spannung von 10 000 V.h isoelektrisch fokussiert. Der pH-Gradient lag zwischen 4,0 und 7,5. Die Auftrennung der Polypeptide nach ihrem Molekulargewicht wurde in der zweiten Dimension innerhalb eines linearen Gradienten von 7,5 - 15% Acrylamid durchgeführt (vgl. D.M. Neville & H. Glossmann, Molecular weight determination of membrane protein and glycoprotein subunits by discontinous gel electrophoresis in dodecylsulfate, Meth. Enzym., **32**, 92-102, (1974)). Zur Abschätzung der Molekulargewichte wurde in der zweiten Dimension ein mit ¹⁴C markierter Protein-Marker (Amersham, GB) aufgetragen. Anschließend wurde das Gel mit Hilfe des autoradiographischen Films Kodak X-AR5 fluorographiert (vgl. W.M. Bonner & R.A. Laskey, A film detection method for Tritiumlabelled proteins and nucleic acids in polyacrylamide gels, Europ. J. Biochem., **46**, 83-88, (1974)). Die Expositionszeit betrug 8 Tage. Die Ergebnisse sind in Figur 2 wiedergegeben.

Diese zeigt das Muster der zellfrei synthetisierten Translationsprodukte, die sich von Nebenhoden- bzw. Hoden-spezifischer Poly(A)⁺RNA ableiten.

a) Für die Erstellung der cDNA-Bibliothek wurde Poly(A)⁺RNA aus dem Nebenhoden-Gewebe eines nicht medikamentös behandelten Patienten eingesetzt (bezüglich Muster der in vitro-Translationsprodukte, vgl. Figur 2b). 20 µg von Poly(A)⁺RNA gemäß Beispiel 1 wurden unter Verwendung von Oligo(dT) als Primer revers transkribiert (vgl. U. Gubler & B.J. Hoffmann, A simple and very efficient method for generating cDNA libraries, Gene, 15, 263-269, (1983)).

Die doppelsträngigen cDNA-Konstrukte wurden auf beiden Seiten mit EcoRI-Linkern ligiert und anschließend in die EcoRI Klonierungsstelle des Bakteriophagen Lambda gt11 (Clontech California) eingeführt.

Bakterien des E. coli-Stammes Y 1090 wurden bis zur logarhithmischen Phase angezogen, in Weich-Agarose überführt und zusammen mit der nicht amplifizierten Lambda-Bibliothek in einer Phagendichte von 500 bis 1000 pfu pro Petrischale (15 cm) ausplattiert und 8 Stunden lang bei 42°C inkubiert.

Für das differentielle Screening wurden die Plaques einer jeden Schale nachfolgend auf zwei Nitrocellulose-Filter (Schleicher & Schüll, Darmstadt, BRD, BA85) Replika-plattiert, wobei die Inkubationszeit für das erste Filter 1 Minute, für das zweite Filter 2 Minuten betrug.

In dieser Weise wurden Replika-Filter von insgesamt 20 Petrischalen hergestellt und anschließend mit jeweils zwei einzelsträngigen, radioaktiv markierten cDNA-Sonden (vgl. b) unten) (positiv/negativ) hybridisiert.

b) Die Sonden aus Poly(A)⁺RNA von Nebenhoden (liefert positive Sonde) und aus Poly(A)⁺RNA von Hoden (liefert negative Sonde) wurden unter Verwendung von Oligo(dT) als Primer wie folgt hergestellt:

Beide RNA-Spezies entstammten den entsprechenden Geweben eines Patienten, der keiner medikamentösen Vorbehandlung ausgesetzt worden war. Jeweils 1 µg der Poly(A)⁺RNA gemäß Beispiel 1 wurde 5 Minuten lang bei 65°C in einem Volumen von 2 µl denaturiert. Nach schnellem Abkühlen der Ansätze auf Eis wurden jeweils folgende Bestandteile nacheinander zugegeben:
- 2 µl Oligo(dT) (100 µg/ml)
- 1 µl dNTP-Mix (dATP, dGTP, dTTP jeweils 2 mM)
- 1 µl 40 mM Natriumpyrophosphat
- 1 µl RNasin (Amersham; GB)
- 2 µl 10x Reverse Transkriptase-Puffer (500 mM Tris.-Cl (pH 8,5), 500 mM KCl, 100 mM MgCl₂, 100 µg/ml BSA, 10 mM EDTA, 10 mM Dithiotreitol)
- 20 Einheiten AMV reverse Transkriptase (Boehringer Mannheim, BRD)
- 10 µl (α-³²P)-dCTP (NEN, 10 µ Ci/µl; S.A. > 3000 Ci/mmol).

Die Reaktionsansätze wurden 15 Minuten lang bei 42°C inkubiert. Nach Zugabe von 1 µl "Chasemix" (enthaltend alle vier dNTP's in einer Konzentration von jeweils 10 mM) wurde die Inkubation für weitere 20 Minuten fortgesetzt. Die Reaktion wurde durch Zugabe von jeweils 1 µl 0,5 M EDTA abgestoppt und die Produkte ohne weitere Reinigung mit Ethanol gefällt. Die mit einer spezifischen Aktivität von > 10⁸ cpm/µg markierten Sonden wurden mit den oben genannten Replika-Filtern parallel hybridisiert. Die Hybridisierung wurde in 5x Denhardt's-Lösung, 4x SET (200 mM Tris (pH 8,0), 20 mM EDTA, 0,6 M NaCl), 0,1% Natriumpyrophosphat und 25 mM Natriumphosphatpuffer (pH 7,0) 72 Stunden lang bei 65°C und einer Konzentration der Radioaktivität von 5 x 10⁶ cpm/ml durchgeführt. Die Filter wurden anschließend in 0,1% SDS, 2x SSC (300 mM Natriumchlorid, 30 mM Natrium₃-Citrat) bei einer Temperatur von 65°C gewaschen.

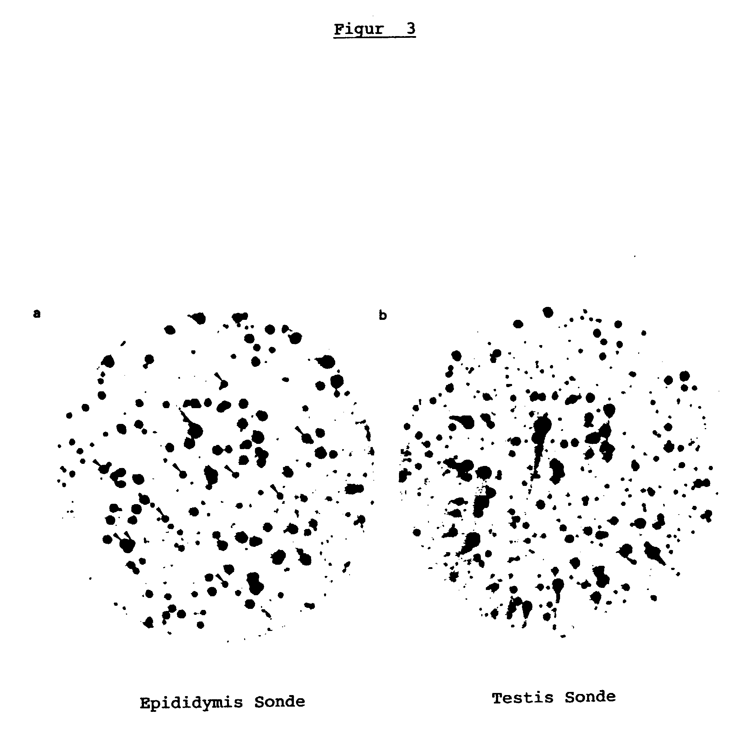

Figur 3 zeigt die Autoradiogramme zweier Replika-Filter von einer der Petrischalen, welche mit epididymalen (a) bzw. testikularen (b) cDNA-Sonden hybridisiert wurden. Die Entwicklung der Autoradiogramme erfolgte nach einer Expositionszeit von 16 Stunden unter Verwendung einer Verstärker-Folie. Die Filter repräsentieren etwa 500 unabhängige Klone der im System Lambda gt 11 erstellten cDNA-Gesamtbibliothek aus Nebenhodengewebe. Die positiven, Epididymis-spezifischen Hybridisierungssignale sind mit Pfeilen markiert (vgl. Figur 3a).

Mit Hilfe dieses Primär-Screening-Verfahrens (vgl. Figur 1a) konnten etwa 10 000 unabhängige cDNA-Klone analysiert werden. Dabei konnten 265 Rekombinanten identifiziert werden, deren Signale nach Hybridisierung mit der epididymalen cDNA-Sonde im Vergleich zu der aus Hodengewebe hergestellten sehr viel stärker waren. Bezogen auf den durch Screening analysierten Teil der Phagen-Bibiothek entspricht die Anzahl positiver cDNA-Klone einem Anteil von 2,5%.

Positive cDNA-Klone wurden isoliert und für ein Sekundär-Screening in 6 Gruppen mit bis zu 50 Klonen aufgeteilt.

Das oben beschriebene Verfahren wurde mit der Abweichung wiederholt, daß von jeder der 6 Petrischalen 3 Replika-Filter angefertigt wurden. Zur Herstellung der negativen cDNA-Sonden wurde hier Poly(A)⁺RNA aus menschlichem Gehirn bzw. aus menschlischer Leber (Clontech California, USA) verwendet.

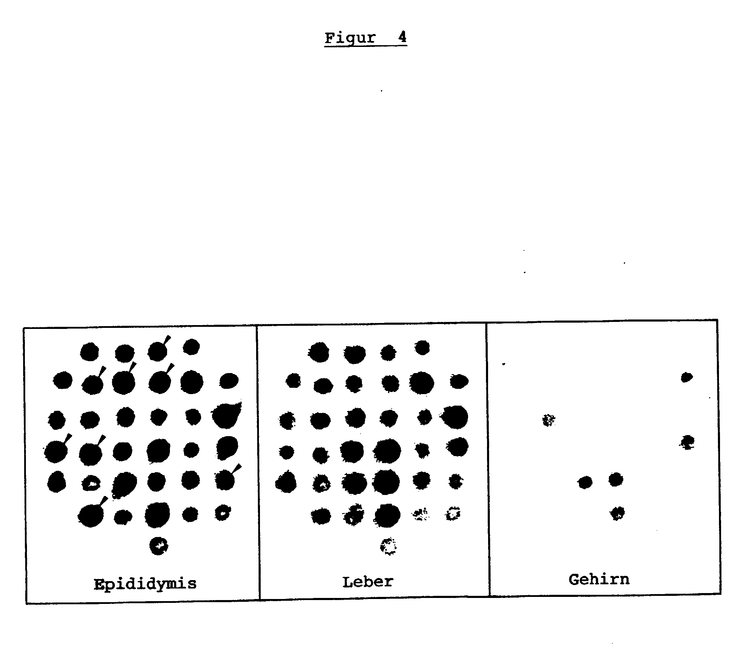

Die Anzahl positiver Rekombinanten wurde nach Analyse dieses sekundären Screening-Verfahrens (vgl. Figur 1b) auf 59 Klone reduziert (vgl. Figur 4).

Die Isolierung positiver Klone erfolgte ohne weitere Reinigung der Plaques. Die gereinigte Lambda-DNA wurde mit EcoRI geschnitten und die Inserts durch Biotrap-Elution (Schleicher & Schüll) isoliert. Die EcoRI-Inserts wurden in den bakteriellen Plasmid-Vektor pBS (Stratagene, California, USA) subkloniert und durch das CaCl₂-Transformationsverfahren in die Bakterienzellen des E.coli-Stammes XL1 Blue (Stratagene, California) eingeführt (vgl. T. Maniatis et al., Molecular cloning: A Laboratory Manual, Cold Spring Harbour Laboratory, Cold Spring Harbour, N.Y., (1982)).

### Beispiel 4

### Herstellung von Northern-Blots zur Analyse der gewebespezifischen Genexpression und Einteilung der Epididymis-spezifischen cDNA-Klone in Familien mit verwandten Sequenzen

20 µg Gesamt-RNA aus menschlicher Epididymis (E) von einem mit Cyproteronacetat behandelten Patienten sowie die gleiche Menge Gesamt-RNA aus menschlichem Hoden (T) und menschlicher Decidua (D) wurden in einem horizontalen 1,3%-igen Formaldehyd-Agarosegel 16 Stunden lang bei einer konstanten Spannung von 25 Volt elektrophoretisch aufgetrennt (vgl. Maniatis et al., Molecular Cloning: A Laboratory Manual, Cold Spring Harbour Laboratory, Cold Spring Harbour, N.Y., (1982) (Figur 3)). Zur Größenbestimmung der RNA's wurde das Gel zusätzlich mit einer RNA-Leiter (0,24-9,5 kB) beladen. Die RNA wurde anschließend durch Kapillar-Blotting in Gegenwart von 20 x SSC auf Nylonmembranen (Hybond N, Amersham) übertragen. Die Blots wurden nachfolgend mit radioaktiven, potentiell Epididymis-spezifischen cDNA-Sonden sukzessive hybridisiert. Die Sonden wurden durch EcoRI-Restriktion der im plasmid pBS subklonierten Lambda gtll-DNA sowie anschließender Biotrap-Elution isoliert und wiesen nach radioaktiver Markierung eine spezifische Radioaktivität von > 10⁹ cpm/µg auf (vgl. A.P. Feinberg und B. Vogelstein, A technique for radiolabelling DNA restriction endonuclease fragments to high specific activity, Analytical Biochemistry, **132**, 6-13, (1983)). Die Hybridisierung erfolgte über Nacht bei einer Radioaktivitätskonzentration von 1-2 x 10⁶ cpm/ml. Nachfolgend wurden die Filter schrittweise unter steigender Stringenz gewaschen, beginnend mit 2 x SSC bei Raumtemperatur bis zu 0,1 x SSC bei einer Temperatur von 65°C. Zur Dehybridisierung wurden die Filter 15 Minuten bei 65°C in Gegenwart von 2 mM Tris (pH 7,5), 1 mM EDTA und 0,1% SDS inkubiert. Die getrockneten Filter wurden auf das Vorhandensein von Sondenmaterial autoradiographisch untersucht, bevor sie im Falle ausbleibender Signale für eine wiederholte Hybridisierung mit einer weiteren Sonde verwendet wurden.

In Figur 5 sind Beispiele für die Autoradiogramme der Northern-Blot-Analysen potentiell positiver cDNA-Klone dargestellt. Die Expositionszeit betrug 20 Stunden. Anhand der unterschiedlichen Hybridisierungsmuster wurden die Klone hinsichtlich ihrer Gewebespezifität qualitativ bewertet.
a) Positiver cDNA-Klon, angezeigt durch Epididymis-spezifische Hybridisierung.
b) Positives, aber nicht Epididymis-spezifisches Hybridisierungsmuster.
c) Positiver cDNA-Klon (*), der auch mit rRNA hybridisiert.
d) cDNA-Klon ohne Gewebe-spezifische Expression.

Mit Hilfe dieses Verfahrens konnte eine Anzahl von 36 epididymalen cDNA-Klonen identifiziert werden. Das spezifische Hybridisierungssignal eines jeden Klon-Inserts mit epididymaler RNA war um mindestens eine Größenordnung stärker als solche mit RNA aus Hoden oder Dezidua (vgl. Figur 5a).

Durch die Ergebnisse aus Kreuz-Hybridisierungen mit der ursprünglichen Phagenbibliothek konnten die 36 Epididymis-spezifischen cDNA-Klone fünf unabhängigen Familien (HE 1, HE 2, HE 3, HE 4 und HE 5) zugeordnet werden, die jeweils einen Satz zueinander verwandter Klone umfassen (vgl. Figur 6 für HE 2 und HE 4). Dabei konnte gezeigt werden, daß sich die fünf cDNA-Klon-Familien von fünf unterschiedlichen, relativ kurzen mRNA-Molekülen ableiten. Ihre ungefähren Längen sowie die anhand der Korrelation von Hybridisierungssignalen mit der Phagenbibliothek ausgezählten Häufigkeiten in bezug auf das Vorkommen einer jeden cDNA-Familiensequenz von HE 1 bis HE 5 sind in der Tabelle 1 dargestellt.

**Tabelle 1**

| Häufigkeit des Auftretens humaner Epididymis-spezifischer cDNA's und ungefähre Länge der mit ihnen korrespondierenden mRNA's | | |
|---|---|---|
| Genprodukt | Häufigkeit¹⁾ | Länge der mRNA (kB)²⁾ |
| HE 1 | 0,5% | 0,9 |
| HE 2 | 0,2% | 0,7 |
| HE 3 | 0,06% | 1,0 |
| HE 4 | 0,05% | 0,7 |
| HE 5 | 0,1% | 0,6 |

| | | |
|---|---|---|
| ¹⁾ Ungefähr 100.000 unabhängige Rekombinaten wurden für jede Sequenz einem Screening-Verfahren unterzogen. | | |
| ²⁾ Zur Längenbestimmung der RNA-Moleküle wurde in der denaturierenden Elektrophorese eine RNA-Leiter mit einer Kettenlänge von 0,24 - 9,5 kB aufgetrennt. | | |

### Beispiel 5

### Kontrolle der Gewebespezifität der Klone HE 1 bis HE 5

Jeweils 8 µg Gesamt-RNA aus Epididymis (E), Decidua (D), Hoden (T), Prostata (P), Niere (K), Hypophyse (Pi) und der Lymphoblastom-Zellinie IM9 (I) wurden gemäß Beispiel 4 aufgetrennt und auf Nylonmembranen übertragen. Zur Kontrolle der Gewebespezifität von HE 5 wurden jeweils 8 µg Gesamt-RNA aus Epididymis (E), Schilddrüse (Th), Hypophyse (Pi), Muskel (M), Niere (K), Nebenniere (A) und der Lymphoblastom-Zellinie IM9 (I) analog aufgetrennt und ebenfalls auf eine Nylonmembran übertragen. Als Sonden wurden (³²P)-markierte cDNA-Inserts aus HE 1(a), HE 2(b), HE 3(c), HE 4(d) und HE 5(e) eingesetzt und mit Northern-Blots gemäß Beispiel 4 hybridisiert.

Die Ergebnisse für HE 1 bis HE 5 sind in Figur 7 dargestellt. Es zeigte sich, daß jeder der fünf cDNA-Klone nur mit der epididymalen RNA ein eindeutiges Hybridisierungssignal liefert.

### Beispiel 6

### Kreuz-Hybridisierungen zur Analyse der Spezies-spezifischen Genexpression

Jeweils 20 µg epididymale Gesamt-RNA aus Mensch (H), Rind (B), Ratte (R), Maus (M), Katze (C) und Hund (D) wurden gemäß Beispiel 4 aufgetrennt und auf Nylonmembranen übertragen. Als Sonden wurden (³²P)-markierte cDNA-Inserts aus HE 1(a), HE 2(b), HE 3(c), HE 4(d) und HE 5(e) eingesetzt und mit Northern-Blots gemäß Beispiel 4 hybridisiert, wobei jeweils der Vertreter mit der größten Kettenlänge eingesetzt wurde. In Figur 8 sind die nach einer Expositionszeit von 8 Stunden entwickelten Autoradiogramme der Northern-Blot-Analyse für die Klone HE 1 bis HE 5 schematisch dargestellt.

Bei dem cDNA-Insert aus dem Klon HE 3 konnte keine Kreuz-Hybridisierung mit tierischen RNA's festgestellt werden (vgl. Figur 8c), während das Insert aus dem Klon HE 2 lediglich eine sehr schwache Kreuz-Hybridisierung mit der Rinder-RNA zeigte (vgl. Figur 8b), wohingegen die Inserts aus den Klonen HE 1 und HE 5 starke Signale mit der epididymalen Hunde-RNA aufwiesen (vgl. Figur 8a, e). Da sich die Signale auch nach Waschen unter stringenten Bedingungen nicht reduzierten, liegen hier hohe Sequenzhomologien vor. Bei dem Insert des Klons HE 4 wurde ein starkes Signal mit epididymaler Rinder- und Hunde-RNA und ein sehr schwaches Signal mit der Katzen-RNA beobachtet (vgl. Figur 8d). Keine der menschlichen Sequenzen hybridisierte mit epididymaler RNA aus der Ratte und der Maus.

### Beispiel 7

### Bestimmmung der Basensequenzen der Epididymis-spezifischen cDNA-Klone HE 1 bis HE 5

Die Basensequenz des jeweils längsten cDNA-Klons aus den Familien HE 1 bis HE 5 gemäß den Beispielen 4, 5 und 6 wurde mit Hilfe der Dideoxy-Methode nach Sanger, F. und Coulson; A.R. "A rapid method for determining sequences in DNA by primed synthesis with DNA polymerase" J. Mol. Biol. 94, 441 (1975) ermittelt. Zu diesem Zweck wurden pBS-Subklone gemäß Beispiel 3 hergestellt und unter alkalischen Bedingungen in Einzelstrang-DNA's überführt.

Die Ergebnisse für die jeweiligen Klone sind im Sequenzprotokoll (Seq. ID-Nrn. 1 bis 5) dargestellt.

### Beispiel 8

### Herstellung von Antigenen über bakterielle Expressionsvektoren zur Induktion von Antikörpern

Die Protein-kodierenden Bereiche der in den gemäß Beispiel 3 hergestellten pBS-Subklonen insertierten cDNA-Klone HE 2 und HE 4 wurden mit Hilfe der Restriktionsendonukleasen Bgl I und PstI (HE 2) bzw. DdeI herausgeschnitten und in getrennten Ansätzen auf üblichem Wege (Sambrook et al., "Molecular Cloning - A Laboratory Manual", 2. Auflage, Cold Spring Harbor Laboratory Press (1989)) in den Expressions-Plasmidvektor pET3 (Rosenberg et al., "Vectors for selective expression of cloned DNAs by T7 RNA Polymerase", Gene, 56, 125-135 (1987)) unter Berücksichtigung der jeweiligen Leseraster umkloniert. Dieser Vektor ermöglicht die Insertion der verschiedenen cDNA-Inserts direkt hinter (stromabwärts) eine für das Enzym T7-Polymerase spezifischen Promotorsequenz.

Nach Transformation dieser Konstrukte in den E. coli Stamm BL21 (DE3), welcher über das Resistenzgen für Tetracyclin und über das Gen für die T7-Polymerase verfügt, erfolgte die Anzucht der Transformanten unter Tetracyclinselektion. Durch Zugabe von Rifampicin wurden alle E. coli eigenen Polymerasen inaktiviert. Da lediglich die T7-Polymerase gegen dieses Antibiotikum resistent ist und die erfindungsgemäßen cDNA-Inserts der Kontrolle des Promotors für die T7-Polymerase unterstanden, wurden primär die erfindungsgemäßen Polypeptide exprimiert.

Die auf diese Weise hergestellten bakteriogenen Polypeptide wurden anschließend aus den Bakterienextrakten gelelektrophoretisch aufgereinigt und direkt als Antigene für eine Immunisierung von Kaninchen und Hühnern verwendet.

### Beispiel 9

### Herstellung von Antigenen über chemische Synthese zur Induktion von Antikörpern

Die Sequenz -Asp-Lys-Glu-Gly- (Seq. ID-Nr. 6) wurde per Computer als mögliches antigenes Epitop des HE 4-Polypeptids (Seq. ID-Nr. 4) identifiziert und zusammen mit den flankierenden Aminosäuren in der Gesamtsequenz -Asn-Asp-Lys-Glu-Gly-Ser-Ala-Pro-Gln-Val-Asn-Ile-Asn-Phe- (Seq. ID-Nr. 7) nach dem Verfahren von Merrifield (J.M. Stewart, "Synthesis and use of neuropeptides", Neuroendocrine Peptide Methodology, ed. P.M. Conn, Academic Press, New York, S. 815-844 (1989)) chemisch synthetisiert, wobei ein Cystein an Position 81 der Sequenz von HE 4 (Seq. ID-Nr. 4) durch ein Alanin ersetzt wurde.

In analoger Weise wurde ein potentiell immunogenes Epitop von HE 1 (Seq. ID-Nr. 1) bestimmt und einschließlich flankierender Aminosäuren in der Gesamtsequenz -Gln-Lys-Asp-Lys-Thr-Tyr-Ser-Tyr-Leu-Pro-Val-Lys-Ser-Glu-Tyr-Pro- (Seq. ID-Nr. 8) chemisch synthetisiert.

Die auf diese Weise hergestellten immunogenen Fragmente der Polypeptide HE 1 und HE 4 wurden anschließend jeweils mit m-Maleimidobenzoicsäure-N-hydroxysuccinamid-ester (M8759, Sigma) (Sambrook et al., "Molecular Cloning - A Laboratory Manual", 2. Auflage, Cold Spring Harbor Laboratory Press (1989)) an Trägersubstanzen (Keyhole-Limpet-Hämocyanin, KLH) gekoppelt und als Antigene für die Immunisierung von Kaninchen zur Erzeugung von Antikörpern eingesetzt.

### Anlage zur Beschreibung

SEQ ID NO: 1

ART DER SEQUENZ: Nucleotid mit ensprechendem Protein

SEQUENZLÄNGE: 816 Basenpaare

STRANGFORM: Einzelstrang

TOPOLOGIE: linear

ART DES MOLEKÜLS: c-DNA zu m-RNA

URSPRÜNGLICHE HERKUNFT ORGANISMUS: Mensch
GEWEBE: Nebenhoden

UNMITTELBARE EXPERIMENTELLE HERKUNFT:
cDNA-Bibliothek aus humanem Nebenhoden

MERKMALE: von 11 bis 87 BP Signalpeptid (S)
von 88 bis 483 BP reifes Peptid (S)
von 788 bis 797 BP Polyadenylierungssignal (S)

EIGENSCHAFTEN: gewebespezifisches Sekretprotein des
humanen Nebenhodens HE1

ggattcccgg atg 13
Met
-19

cgt ttc ctg gca gct aca ttc ctg ctc ctg gcg 66
Arg Phe Leu Ala Ala Thr Phe Leu Leu Leu Ala
-8

ctc agc acc gct gcc cag gcc gaa ccg gtg cag 99
Leu Ser The Ala Ala Gln Ala Glu Pro Val Gln
-1 1 4

ttc aag gac tgc ggt tct gtg gat gga gtt ata 132
Phe Lys Asp Cys Gly Ser Val Asp Gly Val Ile
15

aag gaa gtg aat gtg agc cca tgc ccc acc caa 165
Lys Glu Val Gln Val Ser Pro Cys Pro Thr Gln
26

ccc tgc cag ctg agc aaa gga cag tct tac agc 198
Pro Cys Gln Leu Ser Lys Gly Gln Ser Tyr Ser
37

gtc aat gtc acc ttc acc agc aat att cag tct 231
Val Asn Val Thr Phe Thr Ser Asn Ile Gln Ser
48

aaa agc agc aag gcc gtg gtg cat ggc atc ctg 264
Lys Ser Ser Lys Ala Val Val His Gly Ile Leu
59

atg ggc gtc cca gtt ccc ttt ccc att cct gag 297
Met Gly Val Pro Val Pro Phe Pro Ile Pro Glu
70

cct gat ggt tgt aag agt gga att aac tgc cct 330
Pro Asp Gly Cys Lys Ser Gly Ile Asn Cys Pro
81

atc caa aaa gac aag acc tat agc tac ctg aat 363
Ile Gln Lys Asp Lys Thr Tyr Ser Tyr Leu Asn
92

aaa cta cca gtg aaa agc gaa tat ccc tct ata 396
Lys Leu Pro Val Lys Ser Glu Tyr Pro Ser Ile
103

aaa ctg gtg gtg gag tgg caa ctt cag gat gac 429
Lys Leu Val Val Glu Trp Gln Leu Gln Asp Asp
114

aaa aac caa agt ctc ttc tgc tgg gaa atc cca 462
Lys Asn Gln Ser Leu Phe Cys Trp Glu Ile Pro
125

gta cag atc gtt tct cat ctc taa gtgcctcatt 496
Val Gln Ile Val Ser His Leu ***
132

gagttcggtg catctggcca atgagtctgc tgagactctt 536

gacagcacct ccagctctgc tgcttcaaca acagtgactt 576

gctctccaat ggtatccagt gattcgttga agaggaggtg 616

ctctgtagca gaaactgagc tccgggtggc tggttctcag 656

tggttgtctc atgtctcttt ttctgtctta ggtggtttca 696

ttaaatgcag cacttggtta gcagatgttt aatttttttt 736

tttaacaaca ttaacttgtg gcctctttct acacctggaa 776

atttactctt gaataaataa aaactcgttt gtcttgtaaa 816

SEQ ID NO: 2

ART DER SEQUENZ: Nucleotid

SEQUENZLÄNGE: 736 Basenpaare

STRANGFORM: Einzelstrang

TOPOLOGIE: linear

ART DES MOLEKÜLS: c-DNA zu m-RNA

URSPRÜNGLICHE HERKUNFT ORGANISMUS: Mensch
GEWEBE: Nebenhoden

UNMITTELBARE EXPERIMENTELLE HERKUNFT:
cDNA-Bibliothek aus humanem Nebenhoden

MERKMALE: von 99 bis 164 BP Signalpeptid (S)
165 bis 407 BP reifes Peptid (S)
652 bis 657 BP Polyadenylierungssignal (S)

EIGENSCHAFTEN: gewebespezifisches Sekretprotein des
humanem Nebenhodens HE2

GATGAC 6

AT GTATTTGCAC AAAAATTCTC AGGCACATTA AATTATT 45

GTA AACTGAAGTA AAACCTGGGT GCTTTCTGGC TTGCAG 84

TGCT CTTGGCAGAC ATG AGG CAA CGA TTG CTC CCG 119
Met Arg Gln Arg Leu Leu Pro
-22 -20

TCC GTC ACC AGC CTT CTC CTT GTG GCC CTG CTG 152
Ser Val Thr Ser Leu Leu Leu Val Ala Leu Leu
-10

TTT CCA GGA TCG TCT CAA GCC AGA CAT GTG AAC 185
Phe Pro Gly Ser Ser Gln Ala Arg His Val Asn
-1 1

CAC TCA GCC ACT GAG GCT CTC GGA GAA CTC AGG 218
His Ser Ala Thr Glu Ala Leu Gly Glu Leu Arg
10

GAA AGA GCC CCT GGG CAA GGC ACA AAC GGG TTT 251
Glu Arg Ala Pro Gly Gln Gly Thr Asn Gly Phe
20

CAG CTG CTA CGC CAC GCA GTG AAA CGG GAC CTC 284
Gln Leu Leu Arg His Ala Val Lys Arg Asp Leu
30 40

TTA CCA CCG CGC ACC CCA CCT TAC CAA GTG CAC 317
Leu Pro Pro Arg Thr Pro Pro Tyr Gln Val His
50

ATC TCT CAC CAG GAG GCT CGA GGA CCC TCA TTT 350
Ile Ser His Gln Glu Ala Arg Gly Pro Ser Phe
60

AAG ATC TGC GTG GGC TTT TTA GGG CCT AGA TGG 383
Lys Ile Cys Val Gly Phe Leu Gly Pro Arg Trp
70

GCC AGG GGA TGT TCC ACT GGG AAT TAG ATACCAT 417
Ala Arg Gly Cys Ser Thr Gly Asn ***
80 81

CTG CCGTATGCAG CAAGGGATCT GCAGACTTTT TTTCTG 456

CCAT TCTGGTGAGA AAAAGCGTGA CATTTGCTCT GATCC 495

CTGGA ATAGGTGTTG CGTATCAAAT ACAGATGAAG AAGG 534

AAAAGA GAAACCAGAG ATGGATGGCA GATCTGGGAT CTA 573

AAATATA AGCTCCCGGA AGGCAGGGAT GTTGAAGTAT CC 612

CAAGGGCT TAAAGGAATG TGTGGCTTAT AGTAGGTGTT C 651

AATAAATAT TTGTTGAATG AATTTAGMCC AAAGGTGAAG 700

AGCTGATAAA AGAGATTTTT TTAACTTCAA AAAAAA 736

SEQ ID NO: 3

ART DER SEQUENZ: Nucleotid

SEQUENZLÄNGE: 438 Basenpaare

STRANGFORM: Einzelstrang

TOPOLOGIE: linear

ART DES MOLEKÜLS: c-DNA zu m-RNA

URSPRÜNGLICHE HERKUNFT ORGANISMUS: Mensch
GEWEBE: Nebenhoden

UNMITTELBARE EXPERIMENTELLE HERKUNFT:
cDNA-Bibliothek aus humanem Nebenhoden

MERKMALE: von 411 bis 417 BP Polyadenylierungssignal (S)

EIGENSCHAFTEN: gewebespezifische mRNA des humanen
Nebenhodens HE3

gaattccatt gtagcatgga cgggtatgtt gatagcatag 40

aagacctaaa gatggtagaa cctatcggca actagaaagt 80

ctatgcacat cctcaggtat tggtagagta ttcagtgctt 120

tctaagtagc annccctgcc tccatcaata gtcctaccac 160

tcccctcttg catttatttg tcaatgtttt ccaaatactt 200

agagttatga atagcataat ttcttgatac cataacnnna 240

gcctgtgttg tttcnntgcc tggaatacaa tttttgtctt 280

catttaccta atttactctt atacttttgt cgacattcag 320

ctcatatggc atctgttctt gattaaccca agactttcct 360

gatattgact ctcttgatac atacccaagc tgaacaatct 400

tcccttccct aaataaatta tatgactgca aaaaaaaa 438

SEQ ID NO: 4

ART DER SEQUENZ: Nucleotid mit ensprechendem Protein

SEQUENZLÄNGE: 590 Basenpaare

STRANGFORM: Einzelstrang

TOPOLOGIE: linear

ART DES MOLEKÜLS: c-DNA zu m-RNA

URSPRÜNGLICHE HERKUNFT ORGANISMUS: Mensch
GEWEBE: Nebenhoden

UNMITTELBARE EXPERIMENTELLE HERKUNFT:
cDNA-Bibliothek aus humanem Nebenhoden

MERKMALE: von 27 bis 106 BP Signalpeptid (S)
von 107 bis 401 BP reifes Peptid (S)
von 554 bis 557 BP Polyadenylierungssignal (S)

EIGENSCHAFTEN: gewebespezifisches Sekretprotein des
humanen Nebenhodens HE4

cccctgc accccgcccg gcatagcacc 26

atg cct gct tgt cgc cta ggc ccg cta gcc gcc 59
Met Pro Ala Cys Arg Leu Gly Pro Leu Ala Ala
-30 -20

gcc ctc ctc ctc agc ctg ctg ctg ttc ggc ttc 92
Ala Leu Leu Leu Ser Leu Leu Leu Phe Gly Phe
-9

acc cta gtc tca ggc aca gga gca gag aag act 125
Thr Leu Val Ser Gly Thr Gly Ala Glu Lys Thr
-1 1 3

ggc gtg tgc ccc gag ctc cag gct gac cag aac 158
Gly Val Cys Pro Glu Leu Gln Ala Asp Gln Asn
14

tgc acg caa gag tgc gtc tcg gac agc gaa tgc 191
Cys Thr Gln Glu Cys Val Ser Asp Ser Glu Cys
25

gcc gac aac ctc aag tgc tgc agc gcg ggc tgt 224
Ala Asp Asn Leu Lys Cys Cys Ser Ala Gly Cys
36

gcc acc ttc tgc ctt ctc tgc ccc aat gat aag 257
Ala Thr Phe Cys Leu Leu Cys Pro Asn Asp Lys
47

gag ggt tcc tgc ccc cag gtg aac att aac ttt 290
Glu Gly Ser Cys Pro Gln Val Asn Ile Asn Phe
58

ccc cag ctc ggc ctc tgt cgg gac cag tgc cag 323
Pro Gln Leu Gly Leu Cys Arg Asp Gln Cys Gln
69

gtg gac acg cag tgt cct ggc cag atg aaa tgc 356
Val Asp Thr Asn Cys Pro Gly Gln Met Lys Cys
80

tgc cgc aat ggc tgt ggg aag gtg tcc tgt gtc 389
Cys Arg Asn Gly Cys Gly Lys Val Ser Cys Val
91

act ccc aat ttc tga ggtccagcca ccaccaggct 424
Thr Pro Asn Phe ***
95

gagcagtgag gagagaaagt ttctgcctgg ccctgcatct 464

ggttccagcc cacctgccct cccctttttc gggactctgt 508

attccctctt ggggtgacca cagcttctcc ctttcccaac 552

caataaagta accactttca gcaaaaaaaa aaaaaaaa 590

SEQ ID NO: 5

ART DER SEQUENZ: Nucleotid mit ensprechendem Protein

SEQUENZLÄNGE: 544 Basenpaare

STRANGFORM: Einzelstrang

TOPOLOGIE: Linear

ART DES MOLEKÜLS: c-DNA zu m-RNA

URSPRÜNGLICHE HERKUNFT ORGANISMUS: Mensch
GEWEBE: Nebenhoden

UNMITTELBARE EXPERIMENTELLE HERKUNFT:
cDNA-Bibliothek aus humanem Nebenhoden

MERKMALE: von 45 bis 138 BP Signalpeptid (S)
139 bis 282 BP reifes Peptid (S)
504 bis 509 BP Polyadenylierungssignal (S)

EIGENSCHAFTEN: gewebespezifisches Sekretprotein des
humanem Nebenhodens HE5

gaca gccacgaaga tcctaccaaa 44

atg aag cgc ttc ctc ttc ctc cta ctc acc atc 88
Met Lys Arg Phe Leu Phe Leu Leu Leu Thr Ile
-24 -20

acc ctc ctg gtt atg gta cag ata caa act gga 132
Thr Leu Leu Val Met Val Gln Ile Gln Thr Gly
-10

ctc tca gga caa aac gac tac cag cca aac cag 176
Leu Ser Gly Gln Asn Asp Tyr Gln Pro Asn Gln
-1 1

cag ccc ctc agc atc cag cag cat gag cga ggc 220
Gln Pro Leu Ser Ile Gln Gln His Glu Arg Gly
10 20

att ttc ctt ttc ttc gtg gcc aat gcc ata atc 264
Ile Phe Leu Phe Phe Val Ala Asn Ala Ile Ile
30

cac ctc ttc tgc ttc agt tga ggtgacacgt ctcag 308
His Leu Phe Cys Phe Ser ***
36

cctta gccctgtgcc ccctgaaaca gctgccacca tcact 352

cgcaa gagaatcccc ttcatctttg ggaggggttg atgcc 396

agaca tcaccaggtt gtagaagttt gacaggcagt gcatg 440

gggca acagcaaaat agggggtaa tgatgtaggg gccaa 484

gcagt gccxcagctg ggggaataaa gttacccttg tactg 528

caaaa aaaaaaaaaa 544

SEQ ID NO: 6

ART DER SEQUENZ: AMINOSÄURESEQUENZ
SEQUENZLÄNGE: 4 AMINOSÄUREN
UNMITTELBARE EXPERIMENTELLE HERKUNFT: SEQ ID NO 4
EIGENSCHAFTEN: ANTIGENES EPITOP DES HE 4-POLYPEPTIDS

Asp Lys Glu Gly
1   2   3   4

SEQ ID NO: 7

ART DER SEQUENZ: AMINOSÄURESEQUENZ
SEQUENZLÄNGE: 14 AMINOSÄUREN
UNMITTELBARE EXPERIMENTELLE HERKUNFT: SEQ ID NO 4
EIGENSCHAFTEN: ANTIGENES EPITOP DES HE 4-POLYPEPTIDS MIT
FLANKIERENDEN SEQUENZEN

Asn Asp Lys Glu Gly Ser Ala Pro Gln Val
1               5                   10

Asn Ile Asn Phe
14

SEQ ID NO: 8

ART DER SEQUENZ: AMINOSÄURESEQUENZ
SEQUENZLÄNGE: 16 AMINOSÄUREN
UNMITTELBARE EXPERIMENTELLE HERKUNFT
EIGENSCHAFTEN: ANTIGENES EPITOP DES HE 1-POLYPEPTIDS

Gln Lys Asp Lys Thr Tyr Ser Tyr Leu Pro
1               5                   10
Val Lys Ser Glu Tyr Pro
                15

## Patentansprüche

1. DNA-Sequenzen zur Verwendung bei der Klonierung und Expression von humanen Epididymis-spezifischen Polypeptiden in einer pro- oder eukaryontischen Wirtszelle oder als Diagnostika, welche
a) die im Sequenzprotokoll unter den Seq. ID-Nummern 1 und 3 bis 5 angegebenen Nukleotidsequenzen oder syngene Sequenzen oder Fragmente derselben sowie deren komplementäre Sequenzen aufweisen, oder
b) Nukleotidsequenzen aufweisen, die mit den Proteinkodierenden Bereichen der im Sequenzprotokoll unter den Seq. ID-Nummern 1, 4 und 5 angegebenen Nukleotidsequenzen oder Fragmenten derselben hybridisieren, oder
c) Nukleotidsequenzen aufweisen, die mit Ausnahme der durch die Degeneration des genetischen Codes verursachten Abweichungen, mit den unter a) und b) genannten Sequenzen hybridisieren.

2. DNA-Sequenzen nach Anspruch 1 als Einzel- oder Doppelstränge, **dadurch gekennzeichnet**, daß sie mit einem Marker versehen sind.

3. Verwendung der Sequenzen nach den Ansprüchen 1 oder 2 zur Herstellung von Arzneimitteln zur Diagnose männlicher Infertilität.

4. Vektor-Moleküle, **dadurch gekennzeichnet**, daß sie eine der DNA-Sequenzen nach Anspruch 1 unter Aufrechterhaltung der Fähigkeit zur Replikation in geeigneten Wirtszellen insertiert enthalten.

5. Vektor-Moleküle nach Anspruch 4, **dadurch gekennzeichnet**, daß sie die Sequenzen in solcher Weise insertiert enthalten, daß deren Expression in geeigneten Wirtsorganismen erfolgen kann.

6. Prokaryontische oder eukaryontische Wirtszellen, **dadurch gekennzeichnet**, daß sie mit Vektor-Molekülen nach den Ansprüchen 4 oder 5 transformiert sind.

7. Verfahren zur Herstellung humaner, Epididymis-spezifischer Polypeptide oder von Fragmenten derselben mit gleicher Immunogenität, **dadurch gekennzeichnet**, daß man prokaryontische oder eukaryontische Wirtszellen, die mit einer der DNA-Sequenzen nach Anspruch 1 transformiert sind, unter Bedingungen kultiviert, welche die Expression der jeweiligen DNA-Sequenz erlauben und das jeweilige Expressionsprodukt aus dem Kulturansatz gewinnt.

8. Humane, Epididymis-spezifische Polypeptide, **dadurch gekennzeichnet**, daß sie die im Sequenzprotokoll unter den Seq. ID-Nummern 1, 4 und 5 angegebenen Aminosäure-Sequenzen aufweisen sowie allelische Variationen oder Fragmente dieser Sequenzen mit gleicher Immunogenität.

9. Verwendung der Polypeptide nach Anspruch 8 zur Herstellung von Arzneimitteln zur diagnostischen oder therapeutischen Behandlung männlicher Infertilität.

10. Verwendung nach Anspruch 9, **dadurch gekennzeichnet**, daß die Polypeptide mit einem Marker versehen sind.

11. Verwendung der Polypeptide nach Anspruch 8 zur Herstellung von Arzneimitteln zur Immunsterilisierung von Säugern.

12. Antikörper, **dadurch gekennzeichnet**, daß sie in der Lage sind, eine Immunreaktion mit einem der Polypeptide nach Anspruch 8 einzugehen.

13. Antikörper nach Anspruch 12, **dadurch gekennzeichnet**, daß sie monoklonale Antikörper sind.

14. Antikörper nach Anspruch 12 oder 13, **dadurch gekennzeichnet**, daß sie mit einem Marker versehen sind.

15. Verwendung der Antikörper nach den Ansprüchen 12 bis 14 zur Herstellung von Arzneimitteln zur Diagnose männlicher Infertilität.

16. Verwendung der Antikörper nach den Ansprüchen 12 bis 14 zur Herstellung von Arzneimitteln zur Immunsterilisierung von Säugern.
